# FASCICULE DE BREVET EUROPEEN

(11) **EP 1 071 747 B1**
(45) Date de publication et mention de la délivrance du brevet: **18.08.2004**
(21) Numéro de dépôt: 99920605.5
(22) Date de dépôt: 07.04.1999
(51) Int. Cl.: C12N 1/00

(54) **LIGNEE DE CELLULES IMMORTALISEES DERIVEES DE TISSUS CUTANES HUMAINS NORMAUX**
AUS NORMALEM MENSCHLICHEM HAUTGEWEBE IMMORTALIZIERTE ZELLINIEN
IMMORTALISED CELL LINES DERIVED FROM NORMAL HUMAN SKIN TISSUES

(30) Priorité: 17.04.1998 EP 98201247
(43) Date de publication de la demande: 31.01.2001
(73) Titulaire: SOCIETE DES PRODUITS NESTLE S.A., 1800 Vevey (CH)
(72) Inventeur: BAUR, Markus, CH-1603 Aran (CH)
(74) Mandataire: Becker Kurig Straus
(86) Numéro de dépôt international: PCT/EP1999/002347
(87) Numéro de publication internationale: WO 1999/054435

(56) Documents cités:
- EP-A- 0 780 469
- MILS V ET AL: "1,25-Dihydroxyvitamin D3 and its synthetic derivatives MC903 and EB1089 induce a partial tumoral phenotype reversal in a skin-equivalent system." J INVESTIG DERMATOL SYMP PROC, (1996 APR) 1 (1) 87-93. JOURNAL CODE: C0U. ISSN: 1087-0024., XP002077498 United States
- TINOIS ET AL: "GROWTH AND DIFERENTIATION OF HUMAN KERATINOCYTES ON EXTRACELLULAR MATRIX" ARCHIVES OF DERMATOLOGICAL RESEARCH, vol. 279, no. 4, 1987, pages 241-246, XP002077499

## Description

La présente invention a pour objet de nouvelles lignées cellulaires immortalisées dérivées de tissus cutanés humains normaux présentant des caractères de différentiation améliorés, une nouvelle méthode pour produire ces lignées, et différentes utilisations, notamment dans le cadre de la construction d'une peau artificielle.

### État de la technique

La production de lignées cellulaires immortalisées dérivées de tissus cutanés humains a été décrite antérieurement. En général, les procédés utilisés à cette fin recourent à la transformation de cellules cutanées humaines, par exemple de kératinocytes et de mélanocytes, cultivées in vitro avec des agents qui confèrent la propriété d'immortalisation. L'immortalisation concerne la production de cellules qui peuvent être cultivées pendant des temps prolongés in vitro, théoriquement indéfiniment. Ces cellules sont également désignées sous le nom de lignées cellulaires continues. A l'opposé, les cellules non-immortalisées sont seulement capables de se multiplier pendant un nombre défini de divisions cellulaires in vitro. Les cellules immortalisées sont extrêmement avantageuses car elles fournissent une source stable, potentiellement infinie, de cellules ayant des caractéristiques définies. Des agents classiques pour la production de lignées cellulaires immortalisées et de lignées de cellules cutanées humaines immortalisées comprennent en particulier, par exemple, des virus, des virus recombinants et des plasmides qui contiennent des séquences d'ADN qui confèrent la propriété d'immortalisation.

Le procédé le plus courant pour produire des lignées cellulaires humaines immortalisées comprend probablement l'utilisation de séquences du virus simiesque 40 (SV40) et, plus spécifiquement, de l'ADN d'antigène T (T-Ag) volumineux de SV40 comme agent d'immortalisation. Par exemple, Steinberg *et al.* J. Cell Phys, 123, 117-125 (1985); US4885238 (Reddel *et al.*); US4707448 (Major); Stoner *et al*., Cancer Res., 51,365-371 (1991) ; Chopra *et al.,* In vitro Cell Dev. Biol., 30A, 539-546 (1994) ; Chopra *et al*., In Vitro Cell Dev. Biol., 27A, 763-765 (1991); Christian *et al*., Cancer Res., 47, 6066-6073 (1987) ; Rhim *et al.,* Science, 227, 1250-1252 (1985) ; et Grubman *et al*., Gastrointest. Liver Physiol., 29, G1060-G1070 (1994), font connaître l'utilisation de vecteurs SV40 et de vecteurs contenant la séquence d'antigène T volumineux de SV40 pour produire des lignées cellulaires humaines immortalisées. L'introduction de telles séquences est généralement effectuée par infection au moyen du virus SV40 ou d'un virus hybride adénovirus-12/SV40 ou par transfection de cellules avec un plasmide recombinant contenant la longue répétition terminale du virus de sarcome de Roux et la région précoce Ori-SV40 par coprécipitation en présence de phosphate de strontium (voir Brash *et al*., Mol. Cell Biol., 7, 2031-2034, 1987).

Un autre procédé connu pour la production de lignées cellulaires immortalisées, et en particulier de kératinocytes humains immortalisés, comprend la transfection ou l'infection de cellules avec des séquences d'ADN de virus de papillome humain (HPV). Par exemple, US5376542 (Schlegel) décrit l'immortalisation de cellules épithéliales humaines avec les gènes de E6 et E7 isolés de HPV-16, 18, 31, 33 ou 35 ou avec le gène E7 seul pour produire des lignées cellulaires immortalisées non-tumorigènes. En outre, Barbosa et *al.,* Oncogene, 4, 1529-1532 (1989) ; et Münger *et al*., J. Virol., 63(10):4417-4421 (1989) font connaître l'utilisation des gènes E6 et E7 de HPV-16 et HPV-18 pour produire des kératinocytes humains immortalisés. En outre, Dürst *et al*., Oncogene, 1, 251-256 (1987) décrit l'immortalisation de kératinocytes avec le virus du papillome du type 16.

Cependant, bien que de nombreux groupes aient décrit des lignées cellulaires de kératinocytes immortalisés et leur utilisation dans des essais *in vitro,* les lignées de kératinocytes immortalisés antérieures présentaient habituellement une ou plusieurs propriétés rendant leur utilisation désavantageuse. Par exemple, les kératinocytes immortalisés décrits antérieurement présentaient une ou plusieurs des caractéristiques suivantes : (i) réduction ou perte d'expression de marqueurs de différenciation, par exemple de protéines qui sont exprimées par les kératinocytes différenciés normaux, (ii) caractéristiques de croissance modifiées en culture de tissus, et (iii) formation d'un épithélium stratifié et polarisé ayant un *stratum corneum* para-kératosique.

Pour pallier ces inconvénients, EP780469 (Société des Produits Nestlé) propose une nouvelle méthode d'immortalisation de kératinocytes ou de mélanocytes humains utilisant, *en sus* d'un nouveau milieu de culture, le vecteur pLXSHD+SV40(#328) basé sur le virus SV40, ou bien le vecteur pLXSHD+E6/E7 basé sur le virus du papillome humain 16 (HPV-16). Les cellulaire immortalisée ainsi obtenues conservent l'aptitude à la différenciation et à l'expression de protéines et d'enzymes qui sont exprimées par les kératinocytes ou mélanocytes différenciés normaux même après un taux élevé de passages en culture.

Cependant, malgré les descriptions antérieures, il existe encore un besoin important dans la pratique de disposer de kératinocytes humains immortalisés qui possèdent des propriétés encore améliorées. De telles cellules seraient extrêmement avantageuses pour de nombreuses utilisations, en particulier dans des analyses nécessitant des cellules cutanées très différenciées.

### Résumé de l'invention

A cette fin, la présente invention concerne toute lignée cellulaire de kératinocytes humains immortalisés au moyen de gènes tumorigènes fonctionnels provenant d'au moins du virus SV40 et du virus 16 de papillome humain, caractérisée en ce qu'elle
(1) est non-tumorigénique,
(2) conserve l'aptitude à la différenciation et à l'expression de protéines et d'enzymes qui sont exprimées par les kératinocytes différenciés normaux après un taux élevé de passages en culture de tissus, et
(3) forme un épithélium stratifié et polarisé comportant un *stratum corneum* ortho-kératosique, lorsqu'elle est cultivée en culture organotypique dans un milieu sans sérum et sans couche de cellules nourricières.

La présente invention a aussi pour objectif de fournir un nouveau procédé pour produire des lignées de kératinocytes immortalisées dérivées de tissus cutanés normaux.

Un objectif supplémentaire de la présente invention consiste à proposer des procédés d'utilisation de ces lignées de kératinocytes selon l'invention, par exemple pour des analyses immunologiques, pharmacologiques, photo- et chimiotoxicologiques de réaction cutanée et pour l'expression de gènes hétérologues.

### Description des figures

- La figure 1 représente la construction dérivée du virus SV40, à savoir le plasmide pLXSHD+SV40(#328), qui est utilisée pour immortaliser les kératinocytes de la présente invention.
- La figure 2 représente la construction dérivée du virus papillome virus 16, à savoir le plasmide pLXSHD+E6/E7 qui est utilisée pour immortaliser les kératinocytes de la présente invention.

### Description détaillée de l'invention

La présente invention propose des lignées de kératinocytes immortalisés non-tumorigéniques, c'est à dire qui ne forment pas de tumeurs lorsqu'elles sont injectées sous la peau d'un animal à raison d'au moins 2x10⁶ de cellules par injection, par exemple.

Ces lignées conservent également l'aptitude à la différenciation et à l'expression de protéines de différenciation qui sont exprimées par les kératinocytes normaux même après un taux élevé de passages. L'expression "taux élevé de passages" désigne au moins 10 passages en culture, avantageusement au moins 20 à 30 passages, de préférence au moins 50 passages et théoriquement un nombre infini de passages. Par exemple, les kératinocytes immortalisés pouvant être produits conformément à la présente invention expriment les protéines de différenciation consistant en la kératine K1/10, la kératine K14, l'involucrine, la filaggrine et la loricrine même après un taux élevé de passages en culture de tissus.

Les kératinocytes immortalisés de la présente invention ont un profil de cytochrome p450 (CYP450) qui est similaire, sinon identique, à celui des kératinocytes normaux. Par exemple, les cellules de la présente invention expriment le CYP450 1A1, 2E1, 2C18 et 3A5. En outre, les kératinocytes immortalisés de la présente invention expriment des enzymes de phase II, par exemple la glutation-S-transférase π (GSTπ) d'une manière comparable aux kératinocytes normaux non-immortalisés.

En outre, les kératinocytes immortalisés de la présente invention expriment des protéines et des enzymes impliquées dans l'oxydation cellulaire et les réponses inflammatoires, par exemple la superoxyde-dismutase (SOD), et la collagénase de type 1 et le facteur de nécrose tumorale alpha (TNFα) après traitement avec des esters de phorbol d'une manière similaire ou identique à celle des kératinocytes normaux différenciés. Etant donné ces caractéristiques, ces lignées cellulaires représentent une source reproductible extrêmement intéréssante pour des études immunologiques, pharmacologiques, d'inflammation, photo- et chimiotoxicologique de réactions cutanées.

En outre, les lignées de kératinocytes immortalisés de la présente invention, lorsqu'elles sont cultivées en culture organotypique dans un milieu sans sérum [par example le milieu NR2 de Biofluids Inc., U.S., enrichi avec de l'EGF (5 ng/ml), de la vitamine C (38 µg/ml) et du CaCl₂ (1,5 mM)] et sans couche de cellules nourricières (sans fibroblastes), forment un épithélium stratifié et polarisé comportant des couches superficielles kératinisées, appelées communément *stratum corneum*, ayant une morphologie ortho-kératosique, signifiant que ce *stratum corneum* est dépourvu de cellules nuclées, c'est à dire de cellules contenant leur noyau.

L'obtention d'un épithélium stratifié et polarisé avec des kératinocytes immortalisées avait été obtenu auparavant dans des conditions classiques de culture, c'est-à-dire par une technique utilisant un milieu contenant du sérum de veau et une couche de cellules nourricières (Lechner *et al*., Virology, 185, 536-571. 1991). Cependant, les lignées immortalisées ne formaient pas alors les couches superficielles kératinisées normales. Par exemple, les lignées cellulaires immortalisées avec le virus du papillome 16 ou 18, ou E6/E7, sont connues pour former des épithéliums très désorganisés (Blanton *et al*., Am. J. Pathol., 138, 673-685, 1991; Hudson *et al.,* J Virol., 64, 519-526, 1990; McCane *et al*., Proc. Natl. Acad. Sci., 85, 7169-7173,1988; Woodworth *et al*., Oncogene, 7, 619-626, 1992).

Par contre, les lignées décrites dans EP780469 (Société des Produits Nestlé), lorsqu'elles étaient cultivées en culture organotypique dans un milieu sans sérum et sans couche de cellules nourricières, formaient un épithélium stratifié et polarisé comportant des couches superficielles kératinisées normales, mais ayant cependant une morphologie para-kératosique, signifiant que le *stratum corneum* contenait toujours des cellules avec leur noyau.

Les kératinocytes immortalisés de la présente invention absorbent également des acides gras essentiels (AGE) exogènes et présentent une désaturation et un allongement de chaîne des AGE parfaitement en accord avec ceux des kératinocytes normaux.

En général, les kératinocytes immortalisés de la présente invention peuvent être obtenus par le procédé suivant:
(i) préparation d'un échantillon obtenu d'une peau humaine pour obtenir des kératinocytes primaires aptes à la culture;
(ii) culture de ces kératinocytes primaires dans un milieu sans sérum, de préférence dans le milieu NR-3 (décrit dans EP780469), sur des plaques de culture portant un revêtement qui facilite la fixation et la croissance des cellules, ledit revêtement comprenant de la fibronectine, de la SAB et du collagène de type I;
(iii) remplacement du milieu sans sérum de la manière nécessaire pour parvenir à une croissance confluente optimale des kératinocytes sur les plaques de culture tout en maintenant de manière continue le revêtement sur les plaques de culture ;
(iv) séparation des kératinocytes en culture des mélanocytes, et transfert des kératinocytes séparés dans un milieu d'infection, de préférence le milieu NR-3, et de préférence après traitement des cellules avec une composition contenant la trypsine et de I'EDTA en utilisant des plaques de culture revêtues de manière similaire ;
(v) infection des cellules avec des gènes tumorigènes fonctionnels d'au moins deux virus différents, comme le virus SV40 et le virus 16 de papillome humain ;
(vi) transfert des kératinocytes immortalisés à un milieu de prolifération sans sérum sur des plaques de culture revêtues préalablement de manière similaire, de préférence les milieux NR-2 ou NR-3 (milieux décrits dans EP780469. dont la composition est incorporée par référence à la présente description); et
(vii) transfert des kératinocytes immortalisés ayant proliféré dans un milieu de différenciation convenable ayant une haute teneur en calcium (1,5 mM), de préférence le milieu NR-2 enrichi avec de l'EGF (5 ng/ml) et de la vitamine C (38 µg/ml).

Plus précisément, une étape ayant lieu avant l'étape (i) comprend habituellement l'obtention d'échantillons de tissu cutané humain à partir de donneurs humains normaux, par exemple d'échantillons obtenus au cours d'une intervention chirurgicale ou d'une intervention en pédiatrie. L'immortalisation d'un échantillon de cellules. cutanées unique, c'est-à-dire d'un échantillon de cellules cutanées autologue, permet la production de lignées de kératinocytes immortalisée qui présentent des caractéristiques définies, par exemple un profil de récepteur particulier qui est caractéristique d'un donneur particulier.

L'échantillon de tissu cutané est ensuite préparé dans l'étape (i) de telle sorte qu'il soit apte à la culture in vitro. Cette préparation est effectuée de préférence en lavant initialement l'échantillon de tissu cutané, par exemple en utilisant le milieu qui est utilisé pour la culture. De préférence, cette opération est effectuée dans du milieu NR-2, qui est un milieu sans sérum, dont la composition exacte est décrite dans EP780469, qui s'est révélé être avantageux pour la culture des kératinocytes normaux. Après lavage, l'échantillon de tissu cutané est de préférence rasé, par exemple au moyen d'un dermatome, et est ensuite coupé en petits morceaux.

Les sections cutanées résultantes sont ensuite de préférence séparées en derme et épiderme. Cela peut être réalisé par un moyen physique et/ou un moyen enzymatique. Par exemple, cela peut être réalisé par traitement à la trypsine, par exemple en faisant flotter des échantillons de tissu cutané dans une solution de trypsine (par exemple environ 0,5 %) contenant de l'EDTA (par exemple à environ 0,1 %) pendant un temps suffisant pour provoquer la séparation des cellules, par exemple pendant un temps d'environ 30 à 60 minutes à une température de 37°C ou bien pendant une nuit à 4°C

Le derme est séparé, et l'épiderme est ensuite placé dans un milieu de mise en suspension. De préférence, le milieu de mise en suspension contient une solution d'inhibiteur de trypsine de soja (SBTI), et il est mis en contact avec les cellules pendant un temps suffisant, habituellement d'environ 5 minutes, afin d'inactiver la trypsine et de provoquer la libération des cellules. Un milieu de cul de tissu, de préférence le milieu NR-2 dépourvu de sérum (décrit dans EP780469) et un filtre (par exemple un filtre de 100 mm) sont ensuite ajoutés pour obtenir les cellules désirées, c'est-à-dire les kératinocytes.

Les kératinocytes primaires résultants obtenus dans l'étape (i) sont ensuite utilisés pour ensemencer un milieu sans sérum, de préférence le milieu NR-3 (décrit dans EP780469), à une concentration cellulaire convenable, de préférence d'environ 1,2 x 10⁴ cellules/cm², sur des plaques de culture revêtues préalablement. Cependant, il est possible de faire varier cette concentration de cellules dans de larges limites. Les plaques de culture sont de préférence munies d'un revêtement continu d'une composition qui s'est révélée accroître la fixation et la croissance des kératinocytes, plus précisément une solution de fibronectine, de SAB et de collagène de type I.

Dans l'étape (iii) le milieu de culture est remplacé aussi souvent que nécessaire pour parvenir à une croissance cellulaire optimale. De préférence, le milieu est remplacé environ tous les deux jours. Cependant, cela peut varier en fonction de l'échantillon de tissu cutané particulier. Après avoir atteint une confluence pratiquement totale, par exemple environ 90 % de confluence, ce qui se produit habituellement après un temps d'environ 10 à 14 jours, les kératinocytes et mélanocytes sont séparés. Cela peut être réalisé par n'importe quel moyen qui permet une séparation adéquate des cellules sans effet néfaste sur les mélanocytes et kératinocytes. Par exemple, cela peut être effectué par un traitement différentiel à la trypsine. De préférence, les mélanocytes ou kératinocytes sont traités avec une solution de trypsine/EDTA, puis sont transférés au milieu de sélection. Dans le cas des kératinocytes, les cellules sont de préférence traitées pendant un temps d'environ 5 à 10 minutes avec une solution de trypsine/EDTA (0,025 %/0,01 %) et sont ensuite utilisées à l'étape (iv) pour ensemencer du milieu NR-3 sur des plaques revêtues préalablement.

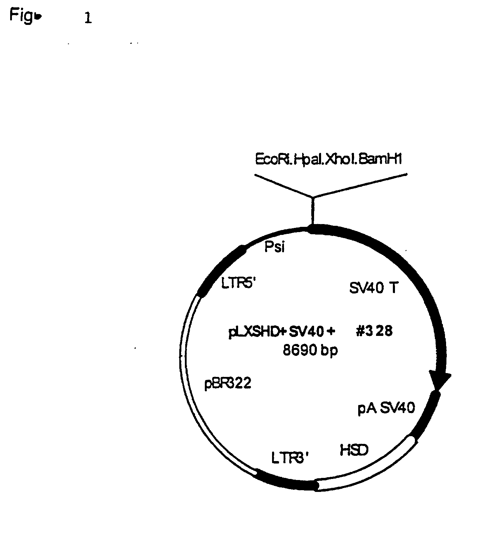

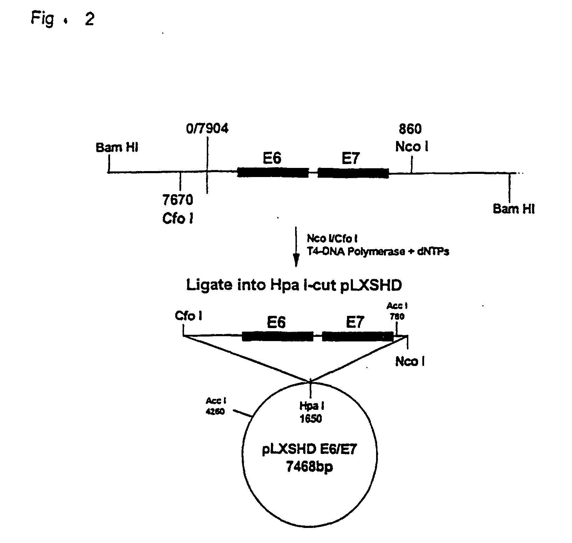

Les kératinocytes sont ensuite traitées avec l'agent d'immortalisation. Les cellules peuvent aussi être congelées jusqu'à ce que l'immortalisation soit effectuée, par exemple dans de l'azote liquide. L'infection et l'immortalisation sont de préférence effectuées en utilisant des gènes tumorigènes fonctionnels d'au moins deux virus différents, comme le T-Ag du virus SV40 et le E6/E7 du HPV16. Ces gènes peuvent être portés chacun dans une construction rétrovirale indépendante, par exemple par le vecteur rétroviral pLXSHD+SV40(#328) qui est représenté à la figure 1 et qui a été décrit par Stockshlaeder *et al.* (GeneBank, n° accession M64753; Human Gen. Therapy, 2, 33-39, 1991), et le vecteur rétroviral pLXSHD+E6/E7 qui est représenté à la figure 2.

Le vecteur rétroviral pLXSHD+SV40(#328) contient la séquence T-Ag de SV40, les séquences de longue répétition terminale 5' et 3' de SV40, les séquences de pBR322 qui permettent la réplication de E. coli, un cycle de clonage multiple, une séquence de polyadénylation de SV40, entre autres séquences.

Le vecteur pLXSHD+E6/E7 contient, à la place du gène codant. pour l'antigène T, le fragment NcoIlCfoI du gène E6/E7 provenant du virus 16 du papillome humain.

Après l'immortalisation, les cellules sont ensuite soumises au nombre de passages nécessaires au cours de la culture et les cellules immortalisées résultantes sont ensuite transférées à un milieu de prolifération dans l'étape (vi). Ce transfert est de préférence effectué au deuxième passage. Ce milieu de prolifération peut être un milieu sans sérum, de préférence le milieu NR-2 ou NR-3. Les cellules immortalisées sont ainsi cultivées sur des plaques de culture revêtues préalablement de manière continue, le revêtement comprenant de nouveau une solution de fibronectine, de SAB et de collagène de type 1.

Après multiplication des cellules immortalisées dans le milieu de prolifération (de préférence NR-2), les kératinocytes sont transférés dans l'étape (vii) dans un environnement qui provoque la différenciation des kératinocytes normaux et immortalisés, de préférence dans un environnement qui simule les conditions retrouvées dans la peau, provoquant ainsi l'organisation des kératinocyte en un épithélium stratifié et polarisé comportant des couches superficielles kératinisées normales. Pour cela, on peut cultiver les cellules dans un milieu sans sérum à haute teneur en calcium, comme le milieu NR-2 comprenant environ 1,5 mM de calcium, environ 5 ng/ml d'EGF et environ 38 µg/ml de vitamine C, la culture étant effectuée sur des plaques pendant 2-3 semaines à l'interphase air-liquide, par exemple sur les plaques de 12 puits Falcon No. 3043, chaque puit comportant un insert Falcon No. 3180 dans lequel les kératinocytes se développent à l'interface air/liquide. L'air est constitué de l'atmosphère contenu dans l'espace interne de l'insert lorsque celui-ci est dépourvu de milieu nutritif. Le liquide est le milieu nutritif contenu dans le puit, ce milieu traversant la membrane de l'insert sur laquelle les kératinoytes se développent.

Eu égard aux propriétés des kératinocytes immortalisés de la présente invention, ceux-ci sont parfaitement aptes à des études immunologiques, pharmacologiques, photo- et chimio-toxicologiques de réactions cutanées. Par exemple, les lignées de kératinocytes immortalisés de la présente invention peuvent être utilisés dans des analyses qui nécessitent des cellules cutanées différenciées, par exemple des études de fonction de barrière (kératinisation) d'un tissu cutané reconstruit, des études de métabolisme des kératinocytes différenciés (métabolisme des acides gras, métabolisme anti-oxydant), des études concernant les effets du rayonnement ultraviolet sur les cellules cutanées, des études concernant les effets d'irritants et sensibilisants cutanés potentiels sur les cellules cutanées, des études de métabolisme des lipides, un traitement topique et/ou par de milieu avec des agents xénobiotiques (par exemple des huiles cosmétiques, en sélectionnant les composés protecteurs éventuels, par exemple des photoprotecteurs), des études d'inflammation et d'irritation cutanée, etc.

En outre, les lignées de kératinocytes produits conformément à la présente invention sont utiles pour sélectionner des composés anti-cancéreux potentiels et des composés potentiels pour le traitement de maladies cutanées. Cela comporte habituellement la mise en contact de la lignée cellulaire avec de tels composés pendant un temps donné et la détermination de l'induction éventuelle de quelconques effets néfastes, par exemple une génotoxicité, une formation d'un produit d'addition d'ADN, une mutagénicité, une transformation cellulaire ou une cytotoxicité.

En outre, les lignées de kératinocytes immortalisés de la présente invention présentent une utilité dans des essais de mutagénèse d'ADN, des essais de sélection d'agents mutagènes cutanés, des essais d'identification d'agents d'altération des chromosomes, des études de transformation maligne, des études de biochimie cellulaire (par exemple des essais d'activation de CYP450), la sélection de composés et de compositions, par exemple de cocktails d'acides gras essentiels qui sont impliqués dans des réactions inflammatoires et allergiques, des essais d'activation de collagénase (en rapport avec l'inflammation), impliquant le TNFα, et la détection d'interleukine.

Eu égard au fait que les lignées selon la présente invention forment un *stratum corneum* ortho-kératosique, celles-ci sont ainsi particulièrement bien adaptées pour participer à la préparation d'une peau artificielle destinée aux études de mutagenicité, immunologiques, pharmacologiques, photo- et chimio-toxicologiques sus-mentionnées. Cette peau peut être constituée uniquement par un épithélium de kératinocytes selon l'invention, mais de préférence comprend aussi du collagène, des fibroblastes, voire même des mélanocytes, de sorte à se rapprocher au mieux de la structure de la peau normale de l'homme, par exemple.

En outre, les lignées de kératinocytes de la présente invention sont aptes à l'expression de protéines recombinantes, par exemple de protéines et polypeptides humains, ainsi qu'à la production d'ARN et d'ADN.

Parmi les lignées de kératinocytes immortalisés produites conformément à la présente invention, seule la lignée DK7-NR a été déposée selon le traité de Budapest, à titre d'exemple, le 19 mars 1998 à la Collection Nationale de Culture de Microorganisme (C.N.C.M.), dont l'adresse est 25 rue de Docteur Roux 75724 Paris, France, et à laquelle a été attribué le numéro de dépôt CNCM I-1996. Ce dépôt a été effectué conformément au Traité de Budapest. Toutes les restrictions concernant la disponibilité de cette lignée cellulaire seront irrévocablement levées lors de la délivrance d'un brevet correspondant à la présente demande ou une autre demande qui revendique le bénéfice de priorité sur cette demande.

D'autres caractéristiques de la présente invention apparaîtront au cours des descriptions suivantes d'exemples de formes de réalisation qui sont fournis à des fins d'illustration de la présente invention et qui ne sont pas destinés à être limitatifs. La manipulation des cellules, la préparation des vecteurs, la transformation de cellules, et toutes les autres procédures techniques sont, en l'absence de précisions contraires, effectués selon les protocoles décrits dans l'ouvrage de Sambrook *et al.* (Molecular Cloning, A Laboratory Manual, Cold Spring Harbor Laboratory Press, U.S.A., 1989).

### Exemple 1 Préparation et caractérisations des lignées

On prélève des tissus cutanés du sein. Après séparation du compartiment dermique et du compartiment épidermique, le derme est coupé en petits fragments de 0,2 x 0,2 mm et est fixé sur une plaque de culture de 6 cm avec du sérum. Du milieu essentiel minimal de Dulbecco (DMEM, 10% de sérum de foetus de veau) est ajouté au bout de 2 à 4 heures. Cette culture d'explant est ensuite mise en incubation jusqu'à ce que la croissance excessive des fibroblastes soit visible. Les cultures de fibroblastes confluentes sont scindées et multipliées pour obtenir des cultures de réserve congelées.

On ensemençe avec les cellules primaires des boîtes de culture qui sont revêtues de manière continue avec un revêtement "cocktail" décrit antérieurement pour les cellules bronchiques (Lechner *et al.,* J. Tiss, Cult. Meth., 9:43-49 (1985)). Après avoir atteint une confluence pratiquement totale, par exemple environ 90 % de confluence, ce qui se produit habituellement après un temps d'environ 10 à 14 jours, les kératinocytes et mélanocytes sont séparés. Pour cela, la culture est traitée avec une solution de trypsine/EDTA (0,025 %/0,01 %) pendant 5 min, puis on récolte les mélanocytes qui se sont séparés en premier des kératinocytes. Les kératinocytes primaires sont ensuites cultivées aux nombres de cellules désirés dans un milieu NR-3 sans sérum au moyen des boîtes de culture revêtues décrites (le milieu NR-3 favorise la croissance des kératinocytes par rapport au mélanocytes.

Puis, on transfecte les cellules d'encapsidation "packaging cell line 3T3-fibroblasts" avec les plasmides pLXSHD+SV40(#328) et pLXSHD+E6/E7 suivant le protocole de Pfeifer *et al.* (Meth. Cell Sci., 17, 83-89, 1995) à la différence près que le virus est collecté après encapsidation dans une lignée cellulaire se développant dans du milieu DMEM à 10 % de sérum bovin foetal. Puis, on infecte les kératinocytes avec les virus de sorte à provoquer une immortalisation. Au cours de l'infection, le milieu sans sérum PC-1 décrit dans l'article de Pfeifer et *al*. est également utilisé.

Après immortalisation, les kératinocytes immortalisées sont transférées dans le milieu de prolifération NR-2 ou NR-3 en utilisant des boites de culture revêtues préalablement. Après prolifération des cellules jusqu'aux nombres de cellules désirés, les cellules sont transférées à un milieu de différenciation convenable pour la culture des kératinocytes normaux et immortalisés (NR-2).

Il est démontré que les kératinocytes immortalisés présentent une croissance cellulaire améliorée à des taux, de passages élevés, qui est comparable à celles décrites pour les lignées cellulaires de EP780469.

L'expression de CYP450 1A1, 1A2, 3A5, 2E1, 2B6, 2A6 et 2D6 est analysée dans les cellules cutanées consistant en kératinocytes normaux et immortalisés par transfert d'empreintes par réaction en chaîne avec l'ADN-polymérase à température ambiante (expression d'ARMm,). Le profil en CYP450 exprimé dans les kératinocytes immortalisés est particulièrement similaire, voir identique, à celui des kératinocytes normaux.

Les lignées cellulaires répondent à l'inducteur de CYP450 consistant en benz(a)pyrène comme les cellules non immortalisées même à des taux élevés de passages

Les marqueurs de différenciation sont analysés au moyen d'anticorps spécifiques contre le T-Ag, l'involucrine, la filaggrine, la loricrine, la vimentine et les kératinea K4, K7, K8, K10/1, K13, K14, K17, K18 et K19. La plus forte capacité de différenciation a pu être démontrée pour la lignée DK7-NR

La glutathion-S-transférase (GST) est analysée par Western-Blot et Nothern-Blot. Toutes les lignées de kératinocytes expriment fortement des ARN messagers pour GSTπ. Le profil d'exposition de GSTα, GSTµ et GSTπ dans les lignées cellulaires est similaire à celui des kératinocytes normaux.

Pour analyser et comparer la désaturation et l'allongement de AGE ajoutés dans des kératinocytes, des kératinocytes immortalisés (et des kératinocytes normaux sont traités avec de l'acide linoléique (LA, 15 µM) et de l'acide a-linolénique (LN, 15 µM). Pour ces expériences, le milieu NR-2 (Biofluids Inc.) déficient en AEG est utilisé. Les cultures cellulaires sont traitées après avoir atteint la confluence et sont passées du milieu à un milieu NR-2 à haute teneur en calcium (1,5 mM). Les cellules sont traitées pendant 4 jours avec les AGE (renouvelés au bout de 2 jours). L'analyse des AGE est effectuée par extraction et séparation des phospholipides par CCM (chromatographie sur couche mince) et quantification des esters méthyliques d'acides gras par CGL (chromatographie gaz-liquide). La formation de la désaturation et des produits d'allongement de LA (20:4n-6 et 22:4n-6) et de LN (20:5n-3, 22:5n-3 et 22:6n-3) a pu être démontrée. Ce profil métabolique était en accord avec celui observé dans les kératinocytes normaux.

Toutes les lignes cellulaires étaient hypodiploïdes, avec la plupart des comptages de chromosomes dans l'intervalle des cellules diploïdes. Des cellules autres que celles des lignées cellulaires analysées n'ont pas été détectées dans les cultures. Ce résultat confirme la pureté des lignées cellulaires et l'absence de contamination celullaire par d'autres sources.

La tumorigénicité des kératinocytes immortalisés est déterminée par injection sous-cutanée (1-2 mio de kératinocytes) dans des souris nues. Les lignées de kératinocytes testées, et notamment la lignée DK7-NR ne sont pas tumorigènes chez les souris nues.

### Exemple 2 Construction d'un épithélium

On prépare du milieu NR2 contenant 750.000 cellules d'une lignées cellulaire selon l'exemple 1, on dépose 0,5 ml de ce milieu dans des inserts Falcon No. 3180, on les place dans les puits de plaques Falcon No. 3043 contenant déjà 2 ml de milieu NR-2 frais, et on cultive les kératinocytes pendant 2 jours dans des conditions favorisant la croissance des kératinocytes. Au troisième jour, on enlève le milieu contenu dans l'insert et on laisse les cellules a l'air libre. On change le milieu dans les puits périodiquement tous les 2 jours avec du milieu NR-2 enrichi avec de l'EGF (5ng/ml), de la vitamine C (38µg/ml) et du CaCl₂ (1,5mM). Après 2 à 3 semaines de culture à l'interphase air-liquide, on collecte l'épithelium ainsi formé, on le fixe dans l'acide picrique et on analyse sa morphologie.

Les résultats montrent que les lignées cellulaires de kératinocytes, notamment la lignée DK7-NR, forment un épithélium stratifié et polarisé comportant des couches superficielles kératinisées *(stratum corneum)*. La couche basale de l'épithélium stratifié et polarisé, appelée communément *stratum basale,* contient des cellules ayant une morphologie cuboïdale identique à celle de cellules normales. Le *stratum corneum* présente une morphologie ortho-kératosique, signifiant qu'il est dépouvu de cellules contenant un noyau. La formation de la couche de cellules ortho-kératosiques n'a jamais été possible avec les autres lignées cellulaires immortalisées connues à ce jour. Par exemple, la lignée DK2-NR (EP780469) forme dans des conditions identiques un *stratum corneum* para-kératosique, signifiant que la couche de cellules cornifiées contient encore toujours des cellules avec un noyau. Seul la morphologie ortho-kératosique du *stratum corneum* reflète la situation normale de la peau humaine. En effet, un *stratum corneum* para-kératosique est caractéristique d'une hypertrophie anormale de l'épithélium conduisant à des désordres, tels qu'un psoriasis ou une néoplasie, par exemple.

### Exemple 3 Test d'irritation

On cultive les lignées cellulaires obtenues à l'exemple 1, notamment la lignée DK7-NR, dans du milieu NR-2. L'induction du "gène de stress" TNFα (facteur de nécrose tumorale alpha) après traitement avec des irritants cutanés, consistant en du PMA (12-myristate-13-acétate de phorbol) et des UV-B (rayons ultraviolets B), est analysée par la méthode de transfert d'empreintes Northern et par des essais biologiques. Les résultats montrent que les lignées cellulaires, et tout particulièrement la lignée DK7-NR, répondent au PMA et aux UV-B et expriment la protéine TNFα même un nombre élevé de passages.

### Exemple 4 Construction d'une peau artificielle

On remplace la membrane d'un insert Falcon No. 3180, par une feuille d'ester benzylique d'acide hyaluronique (Hyaff 11). On ensemence le fond de l'insert avec des fibroblastes primaires humain (0,1x10⁶ cellules dans 0,2 ml de milieu), on laisse reposer pendant 30 min, on remplit l'insert avec du milieu DMEM contenant 10% de sérum foetal de veau, on l'incube à 37°C sous une atmosphère contenant 5% de dioxyde de carbone pendant plusieurs jours, on vide l'insert, et on y dépose 0,5 ml de milieu NR2 contenant 750.000 cellules de la lignée cellulaire DK7-NR, on place les inserts dans les puits de plaques Falcon No. 3043 contenant déjà 2 ml de milieu NR-2 frais, et on cultive les cellules pendant 2 jours dans des conditions favorisant la croissance des kératinocytes. Au troisième jour, on enlève le milieu contenu dans l'insert et on laisse les cellules a l'air libre. On change le milieu dans les puits périodiquement tous les 2 jours avec du milieu NR-2 enrichi avec de l'EGF (5 ng/ml), de la vitamine C (38 µg/ml) et du CaCl₂ (1,5mM). Après 2 à 3 semaines de culture à l'interphase air-liquide, on observe la formation d'une peau artificielle présentant les caractéristiques d'une peau normale.

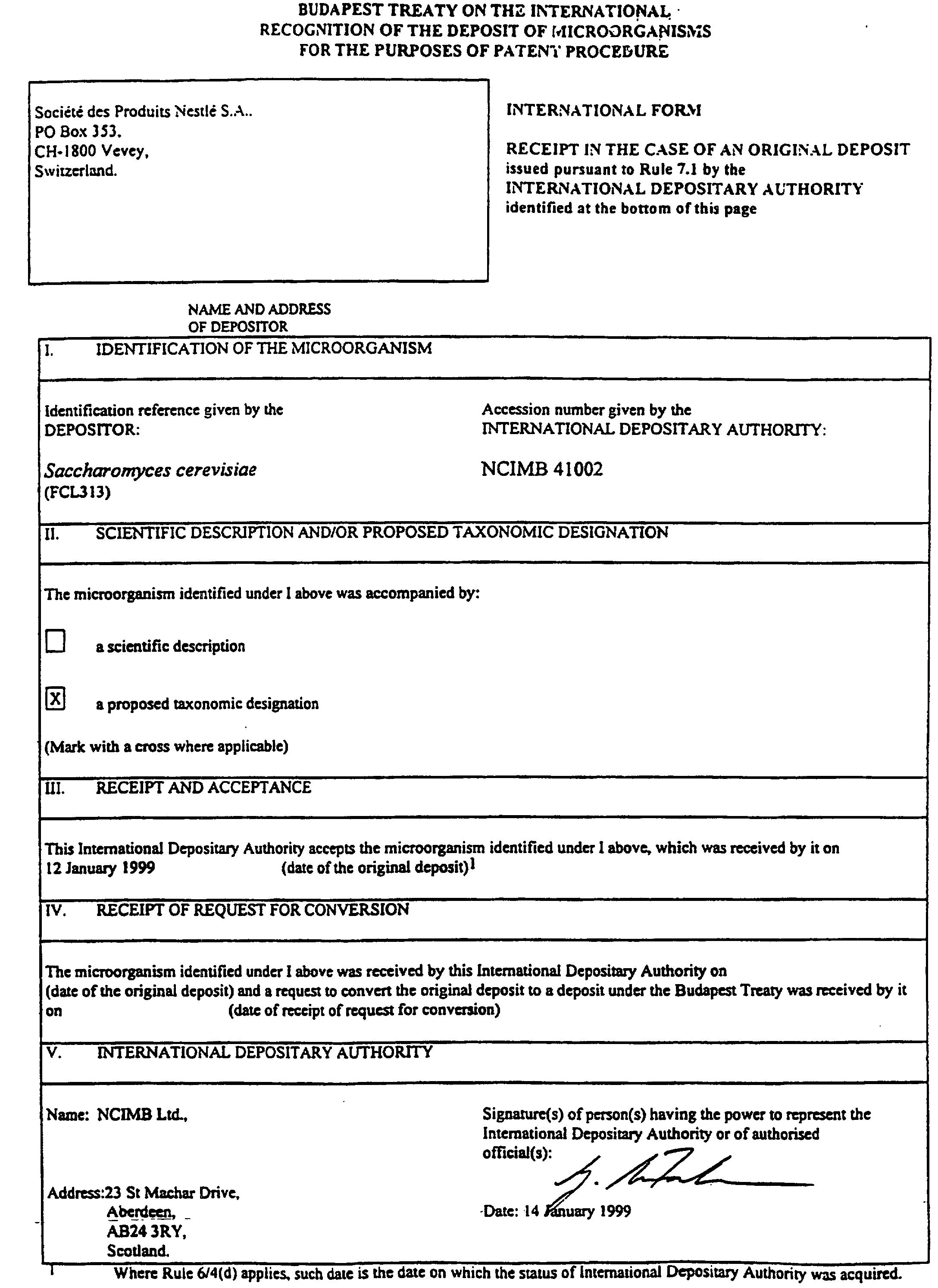
BUDAPEST TREATY ON THE INTERNATIONAL
RECOGNITION OF THE DEPOSIT OF MICROORGANISMS
FOR THE PURPOSES OF PATENT PROCEDURE

Société des Produits Nestlé S.A..
PO Box 353.
CH-1800 Vevey,
Switzerland.

INTERNATIONAL FORM

RECEIPT IN THE CASE OF AN ORIGINAL DEPOSIT
issued pursuant to Rule 7.1 by the
INTERNATIONAL DEPOSITARY AUTHORITY
identified at the bottom of this page

NAME AND ADDRESS
OF DEPOSITOR

I. IDENTIFICATION OF THE MICROORGANISM

Identification reference given by the
DEPOSITOR: | Accession number given by the
INTERNATIONAL DEPOSITARY AUTHORITY:

Saccharomyces cerevisiae
(FCL313) | NCIMB 41002

II. SCIENTIFIC DESCRIPTION AND/OR PROPOSED TAXONOMIC DESIGNATION

The microorganism identified under I above was accompanied by:

[ ] a scientific description

[X] a proposed taxonomic designation

(Mark with a cross where applicable)

III. RECEIPT AND ACCEPTANCE

This International Depositary Authority accepts the microorganism identified under I above, which was received by it on
12 January 1999 (date of the original deposit)1

IV. RECEIPT OF REQUEST FOR CONVERSION

The microorganism identified under I above was received by this International Depositary Authority on
(date of the original deposit) and a request to convert the original deposit to a deposit under the Budapest Treaty was received by it
on (date of receipt of request for conversion)

V. INTERNATIONAL DEPOSITARY AUTHORITY

Name: NCIMB Ltd., | Signature(s) of person(s) having the power to represent the
International Depositary Authority or of authorised
official(s):

Address: 23 St Machar Drive,
Aberdeen,
AB24 3RY,
Scotland. | Date: 14 January 1999

1 Where Rule 6/4(d) applies, such date is the date on which the status of International Depositary Authority was acquired.

BUDAPEST TREATY ON THE INTERNATIONAL
RECOGNITION OF THE DEPOSIT OF MICROORGANISMS
FOR THE PURPOSES OF PATENT PROCEDURE

Société des Produits Nestlé S.A.,
PO Box 353,
CH-1800 Vevey,
Switzerland.

INTERNATIONAL FORM

VIABILITY STATEMENT
issued pursuant to Rule 10.2 by the
INTERNATIONAL DEPOSITARY AUTHORITY
identified on the following page

NAME AND ADDRESS OF THE PARTY
TO WHOM THE VIABILITY STATEMENT
IS ISSUED

I. DEPOSITOR | II. IDENTIFICATION OF THE MICROORGANISM

Name:
AS ABOVE
Address: | Accession number given by the
INTERNATIONAL DEPOSITARY AUTHORITY:
NCIMB 41002

Date of the deposit or of the transfer1:

12 January 1999

III. VIABILITY STATEMENT

The viability of the microorganism identified under II above was tested on 13 January 1999 2. On that date, the said
microorganism was:

3
[X] viable
3
[ ] no longer viable

1 Indicate the date of the original deposit or, where a new deposit or a transfer has been made, the most recent relevant date
(date of the new deposit or date of the transfer).

2 In the cases referred to in Rule 10.2(a)(ii) and (iii), refer to the most recent viability test.

3 Mark with a cross the applicable box.

Form BP/9 (first page)

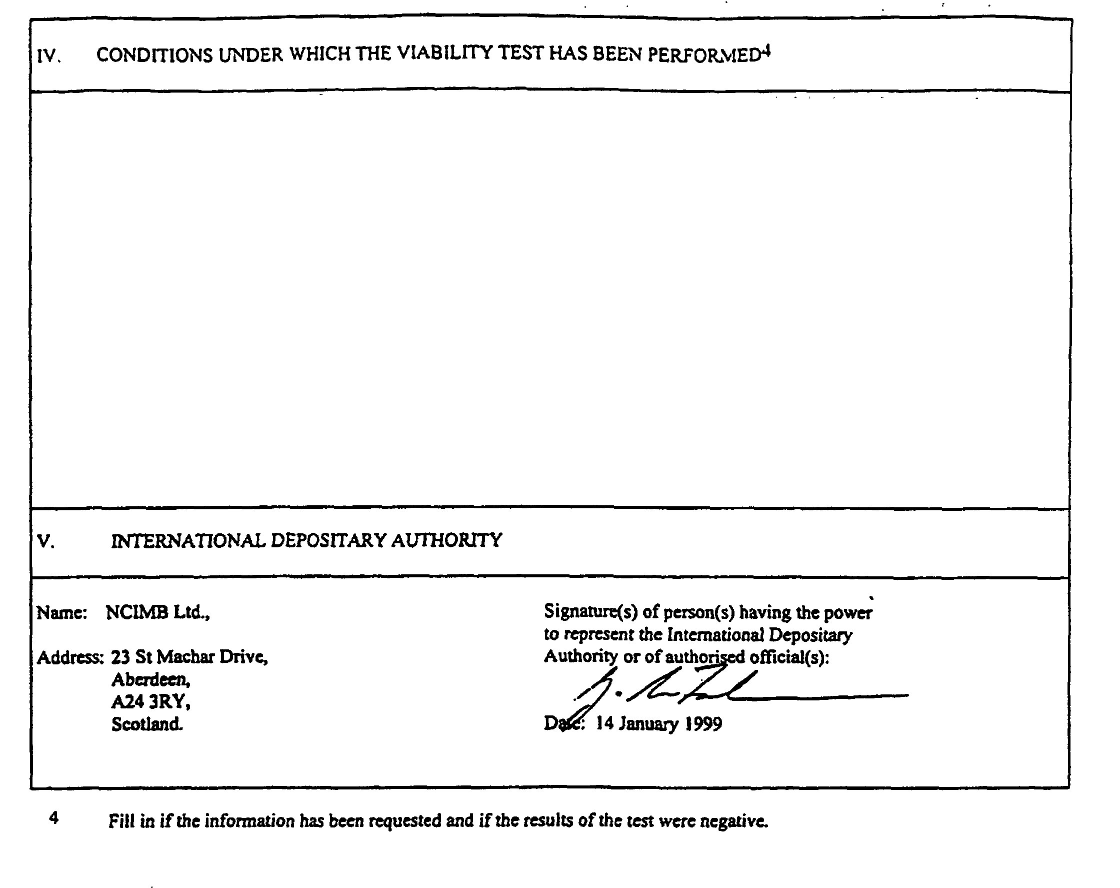
IV. CONDITIONS UNDER WHICH THE VIABILITY TEST HAS BEEN PERFORMED4

V. INTERNATIONAL DEPOSITARY AUTHORITY

Name: NCIMB Ltd., | Signature(s) of person(s) having the power
to represent the International Depositary
Address: 23 St Machar Drive, | Authority or of authorised official(s):
Aberdeen,
A24 3RY, |
Scotland. | Date: 14 January 1999

4 Fill in if the information has been requested and if the results of the test were negative.

## Revendications

1. Lignée cellulaire de kératinocytes humains immortalisés au moyen de gènes tumorigènes fonctionnels provenant d'au moins du virus SV40 et du virus 16 de papillome humain **caractérisée en ce qu'**elle
(1) est non-tumorigénique,
(2) conserve l'aptitude à la différenciation et à l'expression de protéines et d'enzymes qui sont exprimées par les kératinocytes différenciés normaux même après un taux élevé de passages en culture de tissus ledit taux élevé de passages en culture de tissus comprenant au moins 10 passages en culture, et
(3) forme un épithélium stratifié et polarisé comportant un *stratum corneum* orthokératosique, lorsqu'elle est cultivée en culture organotypique dans un milieu sans sérum et sans couche de cellules nourricières.

2. Lignée cellulaire de kératinocytes humains selon la revendication 1, **caractérisée en ce que** chaque gène tumorigène fonctionnel est contenu dans une construction rétrovirale indépendante.

3. Lignée cellulaire de kératinocytes humains selon la revendication 1, caractériséee en qu'il s'agit de la lignée DK7-NR ayant le numéro de dépôt selon le traité de Budapest CNCM I-1996.

4. Procédé perfectionné pour immortaliser des cellules cutanées humaines afin d'obtenir des kératinocytes immortalisés, **caractérisé en ce qu'**il comprend les étapes suivantes:
(i) préparation d'un échantillon obtenu d'un tissu cutané humain pour la culture in vitro;
(ii) obtention de kératinocytes à partir dudit échantillon de tissu cutané préparé et ensemencement avec lesdits kératinocytes d'un milieu de croissance dépourvu de sérum sur des plaques de culture munies d'un revêtement comprenant de la fibronectine, du collagène de type 1 et de la SAB qui facilite la fixation et la croissance des cellules ;
(iii) remplacement du milieu de la manière nécessaire pour parvenir à une croissance confluente optimale des cellules en culture tout en maintenant de manière continue le revêtement sur les plaques de culture;
(iv) transfert des kératinocytes d'un milieu de sélection sans sérum sur des plaques de culture revêtues préalablement de manière similaire;
(v) infection des kératinocytes en utilisant des gènes tumorigènes fonctionnels d'au moins du virus SV40 et du virus 16 de papillome humain;
(vi) transfert des kératinocytes immortalisés résultants à un milieu de prolifération sans sérum convenable pour la prolifération des kératinocytes immortalisés sur des plaques de culture revêtues préalablement de manière similaire; et
(vii) transfert des kératinocytes résultants ayant proliféré à un milieu de différenciation sans sérum, qui contient une haute teneur en calcium sur des boîtes de culture revêtues préalablement de manière similaire.

5. Procédé suivant la revendication 4, **caractérisé en ce que** chaque gène tumorigène fonctionnel est contenu dans une construction rétrovirale indépendante.

6. Procédé suivant la revendication 5, **caractérisé en ce que** les construction rétrovirales utilisées sont les vecteurs pLXSHD+SV40(#328) et pLXSHD+E6/E7, ledit vecteur rétroviral pLXSHD+SV40(#328) contenant la séquence T-Ag de SV40, les séquences de longue répétition terminale 5' et 3' de SV40, les séquences de pBR322 qui permettent la réplication de E. coli, un cycle de clonage multiple, une séquence de polyadénylation de SV40 et autres séquences et ledit vecteur pLXSHD+E6/E7 contenant à la place du gène codant pour l'antigène T, le fragment NcoI/CfoI du gène E6/E7 provenant du virus 16 du papillome humain.

7. Procédé suivant la revendication 4, **caractérisé en ce que** le milieu dépourvu de sérum dans l'étape (ii),(iv) ou (vi) est le milieu NR-3.

8. Procédé suivant la revendication 4, **caractérisé en ce que** le milieu de différenciation dans l'étape (vii) est le milieu NR-2 modifié qui a une teneur en calcium d'au moins 1,5 mM.

9. Utilisation des kératinocytes selon la revendication 1 pour des analyses immunologiques, pharmacologiques, photo- et chimiotoxicologiques de réaction cutanée et pour l'expression de gènes hétérologues.

10. Utilisation selon la revendication 9, **caractérisée en ce que** la lignée DK-7 NR, ayant le numéro de dépôt selon le traité de Budapest CNCM I-1996, est utilisée.

11. Peau artificielle, **caractérisée en ce qu'**elle comprend une lignée de kératinocyte selon la revendication 1.

12. Peau artificielle selon la revendication 11, **caractérisée en ce qu'**elle comprend la lignée DK-7-NR ayant le numéro de dépôt selon le traité de Budapest CNCM I-1996.

## Patentansprüche

1. Menschliche Keratinozyten-Zellinie, immortalisiert mittels funktioneller Tumorgene, die mindestens aus dem SV40 Virus und dem menschlichen Papillomavirus 16 bestehen, **dadurch gekennzeichnet, dass** sie
(1) nicht tumorigenisch ist;
(2) auch nach einer erhöhten Anzahl von Gewebekultur-Passagen die Fähigkeit zur Differenzierung und zur Expression der Proteine und der Enzyme behält, die von differenzierten normalen Keratinozyten exprimiert werden, wobei die Anzahl an Gewebekultur-Passagen mindestens 10 Kultur-Passagen umfassen, und
(3) ein schichtförmiges, polarisiertes Epithel ausbildet, welches ein orthokeratosiches *stratum corneum* umfasst, das in einer organotypischen Kultur in einem Serum-freien Medium und ohne eine Nährzellenschicht gezüchtet wird.

2. Menschliche Keratinozyten-Zellinie nach Anspruch 1, **dadurch gekennzeichnet, dass** jedes funktionelle Tumorgen in einem unabhängigen retroviralen Konstrukt enthalten ist.

3. Menschliche Keratinozyten-Zellinie nach Anspruch 1, **dadurch gekennzeichnet, dass** es sich um den Stamm DK7-NR mit der Hinterlegungsnummer CNCM I-1996 gemäß dem Budapester Abkommen handelt.

4. Verbessertes Verfahren zur Immortalisierung menschlicher Hautzellen zur Gewinnung immortalisierter Keratinozyten, **dadurch gekennzeichnet, dass** das Verfahren die folgenden Schritte umfasst :
(i) Zubereiten einer aus menschlichem Hautgewebe gewonnenen Probe für eine in vitro Kultur;
(ii) Gewinnen von Keratinozyten aus der zubereiteten Hautgewebeprobe und Animpfen eines Serum-freien Wachstumsmediums mit den Keratinozyten auf Kulturplatten, die mit einer Fibronectin, Type 1-Kollagen und BSA umfassenden Beschichtung versehen sind, die die Fixierung und das Wachstum der Zellen fördert;
(iii) Austauschen des Mediums, so dass ein optimales konfluentes Wachstum der Zellen in Kultur erreicht wird, wobei die Beschichtung auf den Kulturplatten weiter aufrechterhalten wird;
(iv) Überführen der Keratinozyten in ein Serum-freies Selektionsmedium auf zuvor in vergleichbarer Weise beschichtete Kulturplatten;
(v) Infizieren der Keratinozyten unter Verwendung von funktionellen, von mindestens dem SV40 und dem menschlichen Papilloma-Virus 16 abgeleiteten Tumorgenen,
(vi) Überführen der so erhaltenen immortalisierten Keratinozyten in ein Serum-freies Proliferations-Medium, das für die Proliferation der immortalisierten Keratinozyten geeignet ist, auf zuvor in gleichartiger Weise beschichtete Kulturplatten, und
(vii) Überführen der so erhaltenen Keratinozyten nach erfolgter Proliferation in ein Serum-freies Differenzierungs-Medium mit hohem Calciumgehalt, auf zuvor in gleichartiger Weise beschichtete Kulturgefässe.

5. Verfahren nach Anspruch 4, **dadurch gekennzeichnet, dass** jedes funktionelle Tumorgen in einem unabhängigen retroviralen Konstrukt enthalten ist.

6. Verfahren nach Anspruch 5, **dadurch gekennzeichnet, dass** die verwendeten, retroviralen Konstrukte die Vektoren pLXSHD+SV40 (#328) und die pLX SHD+E6/E7 sind, wobei der retrovirale Vektor pLXSHD+SV40 (#328) die Sequenz T-Ag des SV40 enthält, die Sequenzen der 5' und 3' langen terminalen Wiederholungen von SV40, die Sequenzen von pBR322, die die Replikation von E. coli, einen mehrfachen Klonierung Zyklus ermöglichen, eine Polyadenylierungs-Sequenz von SV40 und andere Sequenzen, und wobei der Vektor pLXSHD+E6/E7, anstelle des Gens, das das Antigen T kodiert, das NcoI/CfoI-Fragment des Gens E6/E7 des humanen Papilloma Virus 16 enthält.

7. Verfahren nach Anspruch 4, **dadurch gekennzeichnet, dass** das Serum-freie Medium in den Schritten (ii), (iv) oder (vi) das NR-3 Medium ist.

8. Verfahren nach Anspruch 4, **dadurch gekennzeichnet, dass** das Differenzierungs-Medium in Schritt (vii) das modifizierte NR-2 Medium darstellt, mit einem Calciumgehalt von mindestens 1,5 mMol.

9. Verwendung von Keratinozyten nach Anspruch 1 für immunologische, pharmakologische, photo- und chemotoxische Analysen von Hautreaktionen und zur Expression von heterologen Genen.

10. Verwendung nach Anspruch 9, **dadurch gekennzeichnet, dass** der Stamm DK-7NR, mit der Hinterlegungsnummer CNCM I-1996 gemäß dem Budapester Abkommen, verwendet wird.

11. Künstliche Haut, **dadurch gekennzeichnet, dass** sie eine Keratinozyten-Zellinie nach Anspruch 1 enthält.

12. Künstliche Haut nach Anspruch 11, **dadurch gekennzeichnet, dass** sie den Stamm DK7-NR mit der Hinterlegungsnummer CNCM I-1996 gemäß dem Budapester Abkommen enthält.

## Claims

1. A human keratinocyte cell line immortalised by means of functional tumour genes coming from at least SV40 virus and human papilloma virus 16 wherein it
(1) is non-tumorigenic;
(2) conserves the capacity of differentiation and for the expression of proteins and of enzymes expressed by normal differentiated keratinocytes even after an elevated number of passages in culture the said elevated number of passages comprising at least 10 passages in culture; and
(3) forms a stratified and polarised epithelium comprising an ortho-keratosic *stratum corneum* when cultivated in an organo-typical culture in a media without serum and without a layer of nourishing cells.

2. The human keratinocyte cell line according to claim 1, wherein each functional tumorigenic gene is contained in an independent retroviral construct.

3. The human keratinocyte cell line according to claim 1 wherein it is the cell line DK7-NR having the deposit number according to the Budapest Treaty CNCM I-1996.

4. An improved process for immortalising human skin cells to obtain immortalised keratinocytes comprising the following steps:
(i) preparing a sample obtained from a human skin tissue for in vitro culturing;
(ii) obtaining keratinocytes from this prepared sample of skin tissue and seeding a growth medium without serum with said keratinocytes, on culture plates provided with a coating comprising fibronectin, collagen type 1 and BSA, which facilitate the fixation and the growth of cells;
(iii) replacing the medium in a way sufficient to obtain an optimal confluent growth of cells in culture, continuously maintaining the coating of the plate;
(iv) transferring keratinocytes from a selection medium without serum on culture plates coated previously in a similar manner;
(v) infecting keratinocytes using functional tumorigenic genes of at least SV 40 virus and human papilloma virus 16;
(vi) transferring the resulting immortalised keratinocytes into a proliferation medium without serum suitable for the proliferation of immortalised keratinocytes on culture plates coated previously in a similar way;
(vii) transferring the resulting proliferated keratinocytes into a differentiation medium without serum having a high content of calcium on culture boxes coated previously in a similar way.

5. The process according to claim 4, wherein each functional tumour gene is contained in an independent retroviral construct.

6. The process according to claim 5 wherein the used retroviral constructs are the vectors pLXSHD+SV40(#328) and pLXSHD+E6/E7, the said retroviral vector containing the T-Ag of SV40, the long terminal replication sequences 5' and 3' of SV40, the pBR322 sequences which allow the replication of E. coli, a multiple cloning cycle, a polyadenylation sequence of SV40 and other sequences, and the said vector pLXSHD+E6/E7 containing in place of the gene coding for the T-antigen, the NcoI/CfoI fragment of the E6/E7 gene coming from human papilloma virus 16.

7. The process according to claims 4, wherein the medium without serum in step (ii), (iv) or (vi) is the NR-3 medium.

8. The process according to claim 4, wherein the differentiation medium in step (vii) is a modified NR-2 medium having a content of calcium of at least 1,5mM.

9. Use of keratinocytes according to claim 1 for immunological, pharmacological, photo- and chemical-toxicological analysis of skin reaction and for the expression of heterologous genes.

10. The use according to claim9, wherein the line DK7-NR having the deposit number according to the Budapest Treaty CNCM I-1996 is used.

11. Artificial skin comprising a keratinocyte cell line according to claim 1.

12. Artificial skin according to claim 1, comprising the cell line DK7-NR having the deposit number according to the Budapest Treaty CNCM I-1996.
